## Europäisches Patentamt
## European Patent Office
## Office européen des brevets

(11) Publication number: **0 037 172**
**A2**

(12) **EUROPEAN PATENT APPLICATION**

(21) Application number: **81300809.1**

(22) Date of filing: **26.02.81**

(51) Int. Cl.³: **C 07 C 67/08**
C 07 C 69/34, C 07 C 69/80
B 01 J 37/02, B 01 J 23/00
B 01 J 31/04

(30) Priority: **01.03.80 GB 8007064**
**16.04.80 GB 8012530**

(43) Date of publication of application:
**07.10.81 Bulletin 81 40**

(84) Designated Contracting States:
**BE DE FR GB IT NL**

(71) Applicant: **BP Chemicals Limited**
**Britannic House Moor Lane**
**London, EC2Y 9BU(GB)**

(72) Inventor: **Currie, Andrew Christopher**
**BP Chemicals Limited Hayes Road Sully**
**Penarth South Glamorgan CF6 2YU Wales(GB)**

(72) Inventor: **Yeomans, Bertram**
**BP Chemicals Limited Hull Works Saltend**
**Hedon Hull HU12 8DS(GB)**

(74) Representative: **Harry, John et al,**
**BP INTERNATIONAL LIMITED Patents and Licensing**
**Division Chertsey Road**
**Sunbury-on-Thames Middlesex TW16 7LN(GB)**

(54) Heterogeneous esterification catalyst, process for its production and its use in the production of residue esters.

(57) The invention relates to a heterogeneous catalyst comprising one or more metals having an atomic number greater than or equal to twelve in a physical form having a surface area greater than 0.0001 square feet/gram coated with a compound of a metal of Group I to VIII of the Periodic Table other than tin and, optionally, a tin-containing compound. Other aspects of the invention are a process for producing the catalyst and the use of the catalyst in the production of high-boiling esters.

EP 0 037 172 A2

Croydon Printing Company Ltd.

1
## HETEROGENEOUS ESTERIFICATION CATALYST, PROCESS FOR ITS PRODUCTION AND ITS USE IN THE PRODUCTION OF RESIDUE ESTERS.

The present invention relates to novel heterogeneous esterification catalysts, to a process for their production and to their use in the production of high-boiling esters of the type which find use as plasticisers and synthetic lubricants.

Over the years a variety of catalysts has been employed in the production of high-boiling esters, sometimes referred to as "residue esters", which include for example phthalates and stearates. Thus strong mineral acids, such as sulphuric acid and phosphoric acid, and organic acids such as para-toluene sulphonic acid have been used extensively. Notwithstanding their extensive use it has long been recognised that problems are associated therewith. Thus in order to achieve commercially viable production rates at temperatures low enough to produce commercially acceptable products it is necessary to employ such acid concentrations which lead to dehydration of the alcohol reactant giving rise to unwanted olefins and ethers and colouration of the ester products. The colour may be improved in a subsequent process, either physically, by vacuum distillation of the ester product or chemically by an oxidative treatment to convert the colour-forming impurities to substances which may be removed by extraction with an aqueous solution of a strong base.

The problems raised by strong acid catalysts are known to be alleviated to a great extent by the use of amphoteric catalysts. These may be described as compounds of elements capable of functioning as cations or anions as added or subsequently by formation of a complex with the reagents used in the esterification reaction. These
include titanium compounds as described for example in British
Patent Specifications Nos: 852110, 886750, 1058242, 1061173, 1070914
and 1246346. Typical of such titanium compounds are the titanium
esters, e.g. tetraisopropyl titanate. Other amphoteric esterification
catalysts are described in British Patents Specifications
Nos: 879,799 $/\overline{S}b_2O_3\_7$; 733870 $/\overline{A}l(OH)_3$ Na OH$\_7$; 747260 $/\overline{A}l_2(SO)_3/$
NaOH$\_7$; 1076702/$\overline{Z}$nO$\_7$; 1118363 $/\overline{M}oS_2/C\_7$ and 1372854 $/\overline{Z}n(R)_2\_7$.
Another amphoteric esterification catalyst said to be useful in the
production of esters having a lower colour intensity than the products
obtained by the use of other amphoteric catalysts is stannous oxalate
as described in British Patent Specification No: 990297.

A problem associated with the use of some of the amphoteric
catalysts is their lack of solubility in the reaction mixture, it being
recognised in the art that only that portion of the catalyst soluble
in the reaction mixture is catalytically effective. This kinetic
constraint arising from low catalyst solubility and steric hindrance
of the catalyst/reagents complex leads to the use of high temperatures,
generally up to 220$^{\circ}$C, in order to achieve a satisfactory reaction
rate. Quite apart from the economic penalty the use of such high
temperatures can lead to product discolouration.

We have developed active heterogeneous esterification catalysts
by coating metals in filament or particulate form i.e. forms of metal
having very high surface to volume ratios, with a tin-containing
compound. This invention is the subject of our copending application
No: 80/07063 (BP Case No: 4888).

We have now found that many other different metals and non-
metals either alone or in combination with a tin-containing compound
form coatings on metal substrates to produce active heterogeneous
esterification catalysts. Some of these metals and non-metals in
compound form are already known as homogeneous amphoteric esterification
catalysts, others are not so known.

Accordingly the present invention provides a heterogeneous
catalyst suitable for use in the production of esters which catalyst
comprises one or more metals of atomic number greater than or equal to twelve in a physical form having a surface area greater than 0.0001 square feet/gram coated with a compound of a metal of Groups I to VIII of the Periodic Table other than tin and, optionally, a tin-containing compound.

The Periodic Table referred to throughout this specification is that appearing at pages 448/449 of the Handbook of Chemistry and Physics, 44th Edition, published by the Chemical Rubber Publishing Co., which Table includes the Lanthanide series of metals, otherwise known as the "Rare Earths".

Suitable metals of atomic number greater than or equal to twelve may be selected from Groups IB, IIA, IIB, IIIA, IVA, IVB, VA, VB, VIA VIIA and VIII of the Periodic Table. Examples of suitable metals are iron, titanium, copper, aluminium and nickel.

Alloys incorporating one or more of the metals may also be employed. Iron is preferably used as the alloy stainless steel, which contains iron as the major component and chromium and nickel as minor components. Preferably the metal is in the multifilament form. Suitable multifilament forms of the metals copper, titanium, aluminium and stainless steel are commercially available under the trade name "KNITMESH". Such materials are supplied as blanket-type sheets or rolls having a surface area of about 600 square feet/cubic foot. A preferred particulate form of the metal is microspheroidal, e.g. ball bearings.

Suitably the Groups I to VIII metal compound is a carboxylate salt of the metal in which the carboxylate group is derived from a monobasic acid, an aliphatic dibasic acid, a tribasic aliphatic acid a monobasic aromatic acid, a dibasic aromatic acid or a tribasic aromatic acid as hereinafter described in connection with the process for the production of esters.

Suitable metals of Groups I to VIII of the Periodic Table include calcium, cerium, thorium, lead, zinc, aluminium, titanium, magnesium, antimony, manganese, molybdenum and vanadium.

In the case of a coating consisting of a compound of a metal of Groups I to VIII of the Periodic Table and a tin-containing compound the amount of the Groups I to VIII metal compound in the coating should be equal to or greater than 1% by weight based on the weight of the tin-containing compound in the coating.

According to another aspect of the present invention there is provided a process for the production of the catalyst as hereinbefore described which process comprises contacting at elevated temperature one or more metals of atomic number greater than or equal to twelve in a physical form having a surface area greater than 0.0001 square feet/gram with a solution and/or a suspension of a compound of a metal of Groups I to VIII of the Periodic Table other than tin and, optionally, a tin-containing compound in an organic solvent capable of dissolving from 0.001 to 1 gram of the compound or compounds per 100 g of solvent at the temperature of contact.

Suitable compounds which may be used to form the coatings include the oxide and carboxylate salt.

Preferably the solvent is one capable of dissolving from 0.0001 to 0.1 g of the compound or compounds per 100 g of solvent at the temperature of contact. Suitable solvents include esters, ketones and alcohols. The elevated temperature may suitably be greater than $100^{\circ}$C and is preferably in the range from 160 to $300^{\circ}$C.

A very convenient method for preparing the catalyst comprises carrying out an esterification process in the presence of the metal, in filament or particulate form, employing as catalyst an excess of a carboxylate salt of a metal of Groups I to VIII of the Periodic Table other than tin and optionally a carboxylate salt of divalent tin.

Suitably in the preparation of the catalyst the amount of the metal compound or the metal and tin compounds added may be greater than 1.0 g, preferably from 3.0 to 100g of metal or metal and tin ion per square metre of surface area of substrate metal, i.e. metal in filament or particulate form plus metal, if any, forming the surface of the reactor in which the esterification process is carried out.

After carrying out the esterification, thereby coating the metal substrate with a compound of one of the aforesaid metals any excess of that compound may be removed with the ester product.

The present invention also provides a process for the production of an ester boiling above $100^{\circ}$C which process comprises reacting an alcohol with a carboxylic acid or a carboxylic acid anhydride at a temperature greater than 160°C in the presence of an esterification catalyst as hereinbefore described.

The process of the invention is generally applicable to esters which boil above 100°C at atmospheric pressure. It is particularly applicable to esters derived from the following acids:-

monobasic acids - containing up to 20 carbon atoms, e.g. alkanoic acids such as myristic, palmitic and stearic acid, alkenoic acids such as oleic acid or derivatives of such alkanoic and alkenoic acids e.g. ricinoleic acid;

aliphatic dibasic acids - especially those containing up to 20 carbon atoms, preferably up to 10 carbon atoms such as adipic, azelaic or sebacic acid;

tribasic aliphatic acids - such as citric acid;

monobasic aromatic acids - suitably those containing up to 20 carbon atoms, such as benzoic acid;

dibasic aromatic acids and their anhydrides - such as the three phthalic acids especially phthalic acid itself (ortho - phthalic acid) or phthalic anhydride and their hydrogenation products, e.g. hexahydrophthalic anhydride, and

tribasic aromatic acids and their anhydrides - such as hemimellitic, trimellitic or trimesic acids and their anhydrides.

The preferred carboxylic acids are ortho-phthalic acid or its anhydride, adipic acid, azelaic acid or sebacic acid.

The process of the invention is particularly applicable to esters derived from the following alcohols:

monohydric alcohols - containing up to 20 carbon atoms, particularly alkanols containing from 4 to 14 carbon atoms, e.g. butanol, isoheptanol, iso-octanol, 2-ethylhexanol, nonanol, decanol, tridecanol or mixtures of alcohols containing, for example, 7 to 9 carbon atoms such as are obtained from olefinic mixtures by the OXO process;

dihydric alcohols - containing up to 20 carbon atoms, e.g. monoethylene glycol,, diethylene glycol, triethylene glycol, mono-, di- or tripropylene glycol, the butylene glycols or 2,2,4-trimethylpentane diol;

trihydric alcohols - such as glycerol;

aliphatic cyclic alcohols - containing up to 12 carbon atoms such as cyclohexanol;

derivatives - particularly other derivatives of the dihydric and trihydric alcohols, e.g. lower alkyl ether derivatives such as 2-butoxy ethanol.

The preferred alcohols are the monohydric alcohols containing from 4 to 14 carbon atoms.

Preferably there is also added to the esterification reaction mixture a compound which is at least partially soluble in the reaction mixture of the, or each, of the metals in the coating. Preferably the compound is identical to the compound added during formation of the coating. This is believed to be desirable because it is thought that an equilibrium exists between the coating and the reaction medium. The total amount of the soluble compound or compounds added may suitably be greater than 0.005% and preferably between 0.01 and 1.0% by weight of the reaction mixture.

The esterification reaction temperature should be greater than 160°C and preferably is in the range from 180 to 225°C. The temperature may suitably be controlled by the addition of an inert diluent of appropriate boiling point and/or by the use of subatmospheric pressure. Suitable inert diluents include hydrocarbons such as toluene or ortho-xylene, of which ortho-xylene is . amount of inert diluent added may suitably be greater than 5% w/w, preferably from 15 to 30% w/w, based on the weight of the reaction mixture.

In order to take the esterification reaction to completion the amount of alcohol added should be in excess of the stoichiometric amount required to react completely with the acid. The water of reaction may be removed as an azeotrope with excess alcohol, which may be separated and returned to the reaction. Up to a 50% stoichiometric excess, preferably from 10 to 30% stoichiometric excess may be employed. Alternatively or in addition the water of reaction may be removed overhead by the addition of an entrainer which may suitably be the hydrocarbon diluent.

Upon completion of the esterification reaction any unconverted alcohol present in the ester product may be removed by steam-stripping. Alcohol collected in this manner is preferably recycled to the reaction

It is preferred to remove any residual acidity in ester production and to remove any esterification catalyst present therein. This may suitably be achieved by the addition of an inorganic strong base, such as sodium carbonate or lime, which may be added either in the form of a solid or as an aqueous solution. Procedures and conditions for carrying out this step are well-known in the art and typically involve the use of elevated temperature.

The steps of alcohol stripping and neutralisation/catalyst removal are preferably effected outside the reactor in which the esterification process is carried out because the conditions and/or reagents employed have a detrimental effect on the coated metal catalyst. It is therefore preferred to carry out the esterification process in a batch reactor and effect the operations on the ester product batchwise in a separate vessel. The batch reactor in which the esterification process is carried out, may be fabricated in any suitable material such as stainless steel or titanium. Preferably the reactor is fabricated in stainless steel.

The esterification process forming one aspect of the present invention is particularly applicable to the production of plasticisers for use in the plastics industry from, for example, phthalic anhydride and a $C_4$ to $C_{14}$ alkanol or mixture of such alkanols and for the production of esters which are synthetic lubricants.

The invention will now be illustrated by reference to the following Examples.

Preparation of heterogeneous esterification catalysts

Example 1

A mixture of phthalic anhydride (370 g) and 2-ethyl hexanol (780 g) was added to a 2-litre cylindrical reactor lined with 316L stainless steel "KNITMESH" (Viz. two rolls, 10 cm x 5 cm diameter, surface area 8ft$^2$) together with 10 g of zinc oxalate as catalyst. The charge was heated to a reaction temperature of 225$^{\circ}$C with stirring and nitrogen sparging was maintained throughout the heating up and reaction periods.

Water was removed from the reactor during the course of the reaction via a Dean and Stark adaptor, overhead reflux being maintained by reducing the pressure in the system from 0.9 to 0.2 bar during the course of the reaction. The rate of the reaction was followed by monitoring the change in acidity of the reaction mixture.

The results (based on the time required to achieve the stated conversion of phthalic anhydride to ester) are given in Table 1.

Example 2

The procedure of Example 1 was followed except that lead oxalate was used instead of zinc oxalate as catalyst.

Example 3

The procedure of Example 1 was followed except that manganese oxalate was used instead of zinc oxalate as catalyst.

Comparison Test

The procedure of Example 1 was followed except that no metal compound was added as catalyst.

This is not an example according to the invention and is included only for the purpose of comparison.

Table 1

| Example | Comp. Test | 1 | 2 | 3 |
|---|---|---|---|---|
| Periodic Table Group Classification. | | IIb | IVa | VIIb |
| Metal Compound used | None | Zinc Oxalate | Zinc Oxalate | Manganese Oxalate |
| Result Esterification Time (h) | 40 | 15 | 5.5 | 9 |
| Conversion of PA to DOP (%) | 99.8 | 99.8 | 99.97 | 99.95 |

Example 4

The procedure of Example 1 was followed except that the esterification reaction temperature was reduced to 190°C and zinc oxalate was replaced by calcium oxalate as catalyst.

The results (based on the time required to achieve the stated conversion of phthalic anhydride to ester) are given in Table 2.

Example 5

The procedure of Example 4 was repeated except that zinc acetate was used in place of calcium oxalate as catalyst.

Example 6

The procedure of Example 4 was repeated except that aluminium acetate was used in place of calcium oxalate as catalyst.

Example 7

The procedure of Example 4 was repeated except that titanium oxalate was used in place of calcium oxalate as catalyst.

Example 8

The procedure of Example 4 was repeated except that bismuth oxalate was used in place of calcium oxalate as catalyst.

Example 9

The procedure of Example 4 was repeated except that molybdenum trioxide was used in place of calcium oxalate as catalyst.

Example 10

The procedure of Example 4 was repeated except that cerium oxalate was used in place of calcium oxalate as catalyst.

Example 11

A mixture of phthalic anhydride (370 grams) and 2-ethyl-hexanol (781 grams) was added to a 2 l cylindrical, 845-Ti stainless steel reactor lined with, 316L stainless steel "KNITMESH" (two rolls 10 cm x 5 cm diameter, surface area 8 ft.$^2$), together with a mixture (2$^0$ g) of equal weights of stannous oxalate and zinc oxalate. The charge was heated to 190$^0$C with stirring and nitrogen sparging was maintained throughout the heating up and reaction periods.

Water was removed from the reactor during the course of the reaction via a Dean and Stark adaptor, overhead reflux being maintained by reducing the pressure in the system from 0.9 to 0.2 bar during the course of the reaction. The rate of the reaction was followed by monitoring the change in acidity of the reaction mixture.

The reaction mixture was subsequently cooled to ambient temperature prior to discharging the reactor contents, the "KNITMESH" packing being washed several times with the crude ester product to remove any trapped suspension of excess metal salt. The coating obtained comprised 40% and 44% of the stannous and zinc oxalate additions respectively.

Table 2

| Example | 4 | 5 | 6 | 7 | 8 | 9 | 10 |
|---|---|---|---|---|---|---|---|
| Periodic Table Group Classification | IIa | IIb | IIIa | IVb | Va | VIb | Rare Earth |
| Metal Compound Used | Calcium Oxalate | Zinc Acetate | Aluminium Acetate | Titanium Oxalate | Bismuth Oxalate | Molybdenum Trioxide | Cerium Oxalate |
| Result Esterification Time (h) | 10 | 10 | 10 | 5 | 10 | 10 | 12 |
| Conversion of PA to DOP (%) | 97.2 | 98.9 | 97.7 | 99.97 | 99.95 | 97.2 | 97.9 |

Example 12

A mixture of phthalic anhydride (370 g) and 2-ethyl-hexanol (781 g) was added to a 2l cylindrical, 845-Ti stainless steel reactor lined with 316L stainless steel "KNITMESH" (two rolls 10cm x 5 cm diameter, surface area 8 ft.$^2$) together with a mixture of 10 g each of stannous oxalate and manganese oxalate. The charge was heated to 225$^o$C with stirring and nitrogen sparging was maintained throughout the heating up and reaction periods.

Water was removed from the reactor during the course of the reaction via a Dean and Stark adaptor, overhead reflux being maintained by reducing the pressure in the system from 1.0 to 0.6 bar during the course of the reaction. The rate of the reaction was followed by monitoring the change in acidity of the reaction mixture.

The resulting catalyst coating comprised 32% and 15% of the stannous and manganese oxalate additions, respectively.

Example 13

A batch esterification following the procedure of Example 12 was carried out except that manganese oxalate was replaced by boric acid (10 g). A 99.9% conversion of phthalic anhydride to ester was achieved after 7.3 h.

Batch esterification using a coated metal catalyst

Example 14

A mixture of phthalic anhydride (370 grams) and 2-ethyl-hexanol (781 grams) was added to a 2l cylindrical, 845-Ti stainless steel reactor lined with the coated "KNITMESH" obtained in Example 11 together with stannous oxalate (0.148 g) and zinc oxalate (0.03g). The charge was heated to 190$^o$C with stirring and nitrogen sparging was maintained throughout the heating up and reaction periods.

Water was removed from the reactor during the course of the reaction via a Dean and Stark adaptor, overhead reflux being maintained by reducing the pressure in the system from 1.0 to 0.6 bar during the course of the reaction. The rate of the reaction was followed by monitoring the change in acidity of the reaction mixture.

A 99.98% conversion of phthalic anhydride to ester was achieved after 4.5 h.

## Example 15

On completion of Example 12 the product was decanted off, a 'heel' (136.5 grams) including undissolved solids being retained in the reactor. A further charge of reactants, together with zinc oxalate (o.5 gram) was added to the reactor (for incorporation in the catalyst coating) and a further batch esterification was carried out at 180°C following the procedure of Example 3. A 99.96% conversion of PA to ester was achieved in 8.25 hours.

## Example 16

A batch esterification following the procedure of Example 12 was carried out, using the coated "KNITMESH" obtained in Example 15 together with small make-up additions of stannous oxalate (0.148 gram), manganese oxalate (0.03 gram) and zinc oxalate (0.03 gram) to compensate for any loss of metal ion catalyst leaving the system in solution with the ester product.

A 99.96% conversion of PA to ester was thus achieved after 5.5. h at 190°C.

Claims:

1. A heterogeneous catalyst suitable for use in the production of esters which catalyst comprises one or more metals of atomic number greater than or equal to twelve in a physical form having a surface area greater than 0.0001 square feet/gram coated with a compound of a metal of Groups I to VIII of the Periodic Table other than tin and, optionally, a tin-containing compound.

2. A catalyst according to claim 1 wherein the metal of atomic number greater than or equal to twelve is iron, titanium, copper, aluminium or nickel.

3. A catalyst according to claim 1 or claim 2 wherein the form of the metal having a surface area greater than 0.0001 square feet/gram is the multifilament form.

4. A catalyst according to any one of claims 1 to 3 wherein the metal of Groups I to VIII of the Periodic Table is calcium, cerium, thorium, lead, zinc, aluminium, titanium, magnesium, antimony, manganese, molybdenum or vanadium.

5. A catalyst according to any one of the preceding claims wherein the Groups I to VIII metal compound is a carboxylate salt.

6. A process for the production of a heterogeneous catalyst as claimed in any one of claims 1 to 5 which process comprising contacting at elevated temperature one or more metals of atomic number greater than or equal to twelve in a physical form having a surface area greater than 0.0001 square feet/gram with a solution and/or a suspension of a compound of a metal of Groups I to VIII of the Periodic Table other than tine and, optionally, a tin-containing compound in an organic solvent capable of dissolving from 0.0001 to 1 gram of the compound or compounds per 100g of solvent at the temperature of contact.

7.  A process according to claim 6 wherein an esterification process is carried out in the presence of the metal in filament or particulate form employing as catalyst an excess of a carboxylate salt of a metal of Groups I to VIII of the Periodic Table other than tin and, optionally, a carboxylate salt of divalent tin.

8.  A process according to claim 6 wherein the amount of the metal compound or the metal and tin compounds added is in the range from 3.0 to 100g of metal ion or metal and tin ions per square metre of surface area of metal to be coated.

9.  A process for the production of an ester boiling above 100°C which process comprises reacting an alcohol with a carboxylic acid or a carboxylic acid anhydride at a temperature greater than 160°C in the presence of an esterification catalyst as claimed in any one of claims 1 to 5.

10.  A process according to claim 9 wherein the carboxylic acid is orthophthalic acid, adipic acid, azelaic acid or sebacic acid.

11.  A process according to claim 9 or claim 10 wherein the alcohol is a monohydric alcohol containing from 4 to 14 carbon atoms.

12.  A process according to any one of claims 9 to 11 wherein there is also added to the esterification reaction mixture a compound which is at least partially soluble in the reaction mixture of the, or each, of the metals in the coating.

13.  A process according to claim 12 wherein the total amount of the soluble compound or compounds added is between 0.1 and 1.0% by weight of the reaction mixture.

14.  A process according to any one of claims 9 to 13 wherein the temperature is in the range 180 to 225°C.

15.  A process according to any one of claims 9 to 14 wherein the amount of alcohol added is in excess of the stoichiometric amount required to react completely with the acid.